(19) Europäisches Patentamt
European Patent Office
Office européen des brevets

(11) Veröffentlichungsnummer: **0 068 259**
**A1**

(12) **EUROPÄISCHE PATENTANMELDUNG**

(21) Anmeldenummer: **82105170.3**

(22) Anmeldetag: **14.06.82**

(51) Int. Cl.³: **C 07 D 213/74**, C 07 D 407/04, A 61 K 31/44

(30) Priorität: **24.06.81 DE 3124673**

(43) Veröffentlichungstag der Anmeldung: **05.01.83**
**Patentblatt 83/1**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Rosentreter, Ulrich, Dr., Kondorweg 23, D-5600 Wuppertal 1 (DE)**
Erfinder: **Puls, Walter, Dr., In den Birken 75, D-5600 Wuppertal 1 (DE)**
Erfinder: **Bischoff, Hilmar, Dr., Wilkhausstrasse 107, D-5600 Wuppertal 2 (DE)**

(54) **Substituierte 2-Amino-pyridinderivate, Verfahren zu ihrer Herstellung, ihre Verwendung in Arzneimitteln, sowie deren Herstellung.**

(57) Die vorliegende Erfindung betrifft neue in 4-, 5- und 6-Position substituierte 2-Amino-pyridinderivate der allgemeinen Formel I

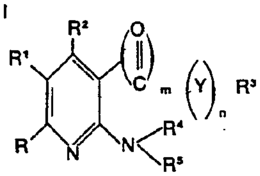

(I)

ein Verfahren zu ihrer Herstellung sowie ihre Verwendung als Arzneimittel, insbesondere ihre Verwendung als Lipidabsorptionshemmer.

EP 0 068 259 A1

0068259

BAYER AKTIENGESELLSCHAFT 5090 Leverkusen, Bayerwerk

Zentralbereich
Patente, Marken und Lizenzen KS/Kü-c
Ia (Pha)

# Substituierte 2-Amino-pyridinderivate, Verfahren zu ihrer Herstellung, ihre Verwendung in Arzneimitteln, sowie deren Herstellung

Die vorliegende Erfindung betrifft neue in 4-, 5- und 6-Position substituierte 2-Amino-pyridinderivate, ein Verfahren zu ihrer Herstellung sowie ihre Verwendung als Arzneimittel, insbesondere ihre Verwendung als Lipidabsorptionshemmer.

Es sind bereits Pyridinderivate mit hypolipidemischer Wirkung bekannt (vgl. Europäische Offenlegungsschrift 0013153). Bei Kenntnis des Standes der Technik war nicht vorhersehbar, daß Pyridinderivate, die in 2-Position einen stickstoffhaltigen Rest tragen und auch in 4-, 5- und 6-Position substituiert sind, vorteilhafte lipidabsorptionshemmende Wirkung besitzen.

Die vorliegende Anmeldung betrifft neue substituierte 2-Amino-pyridinderivate der allgemeinen Formel (I)

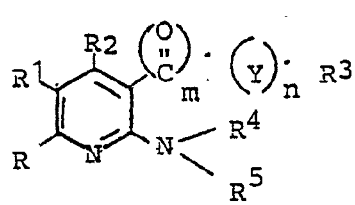

(I)

in welcher

R für Alkyl, gegebenenfalls substituiert durch Hydroxyl, Aryl oder Aralkyl bedeuten,

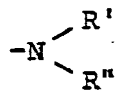

wobei R' und R" gleich oder verschieden sind und jeweils Wasserstoff, Alkyl oder Aralkyl bedeuten, steht
oder für gegebenenfalls substituiertes Aralkyl steht
oder für Aryl steht, welches gegebenenfalls durch 1 oder 2 gleiche oder verschiedene Substituenten aus der Gruppen Halogen, Alkyl, Alkoxy, Alkylmercapto oder Trifluormethyl substituiert ist,

und

R und $R^1$ gemeinsam gegebenenfalls einen carbocyclischen Ring formen, der gegebenenfalls substituiert ist durch Hydroxy, Halogen, Nitro, Alkoxy, die Gruppe

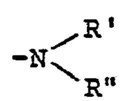 ,

wobei

R' und R" die oben angeführten Bedeutungen haben,

und an den ein Arylring ankondensiert sein kann, wobei der Arylring gegebenenfalls substituiert ist durch 1 oder 2 gleiche oder verschiedene Substituenten aus der Gruppe Halogen, Alkyl, Alkoxy, Alkylmercapto oder Trifluormethyl,

und der gegebenenfalls durch Sauerstoff, Schwefel oder die $NR^6$-Gruppe unterbrochen ist wobei $R^6$ die für $R^1$ und $R^2$ angegebene Bedeutung besitzt, und

$R^1$ und $R^2$ gleich oder verschieden sind und jeweils für Alkyl, gegebenenfalls substituiert durch Aryl, Heteroaryl, Hydroxyl, Halogen, Alkoxy, Carboxy, Cyano, Carbalkoxy oder die Gruppe

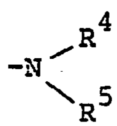

mit den unten angeführten Bedeutungen für $R^4$ und $R^5$ stehen,

oder

für einen Arylrest oder für einen Heteroarylrest aus der Gruppe Thienyl, Furyl, Pyrryl, Pyrazolyl, Imidazolyl, Oxazolyl, Isooxazolyl, Thiazolyl, Pyridyl, Pyridazinyl, Pyrimidyl, Pyrazinyl, Chinolyl, Isochinolyl, Indolyl, Benzimidazolyl, Chinazolyl oder Chinoxalyl stehen, wobei der Arylrest sowie die Heteroarylreste gegebenenfalls 1, 2 oder 3 gleiche oder verschiedene Substituenten aus der Gruppe Phenyl, Alkyl, Alkenyl, Alkinyl, Alkoxy, Alkenoxy, Alkinoxy, Alkylen, Dioxyalkylen, Halogen, Acyloxy, Trifluormethylthio, Trifluormethyl, Trifluormethoxy, Hydroxy, die

Gruppe 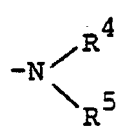

mit den unten angegebenen Bedeutungen für $R^4$ und $R^5$, Nitro, Cyano, Azido, Carboxy, Carbalkoxy, Carbonamido,

Sulfonamido oder $SO_m$-Alkyl (m = 0, 1 oder 2) enthalten, und wobei die Alkyl- und Alkoxysubstituenten ihrerseits gegebenenfalls substituiert sind durch Hydroxy, Alkoxy, Carboxy, Carbalkoxy, Halogen, die Gruppe

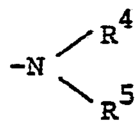 , mit den unten angegebenen Bedeutungen für $R^4$ und $R^5$, Aroyloxy oder Alkanoyloxy, wobei die Aroyl- und Alkanoylreste ihrerseits gegebenenfalls durch Hydroxy, Alkoxy, Halogen, Carboxy oder Carbalkoxy substituiert sind, und

m und n gleich oder verschieden sind und jeweils 0 oder 1 bedeuten, und

Y für Sauerstoff, die Gruppe $SO_{m'}$ (m' = 0,1,2) oder die Gruppe $N-R^6$, wobei $R^6$ einen Alkyl- oder Arylrest bedeutet, steht und

$R^3$ für Wasserstoff oder Alkyl, gegebenenfalls substituiert durch Hydroxy, Epoxy, Alkoxy, Halogen oder die Gruppe 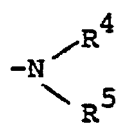

mit den unten angeführten Bedeutungen für $R^4$ und $R^5$ steht, oder für Carboxy, Carbalkoxy, Cyano, Alkenyl, Aryl oder für Aryl, gegebenenfalls substituiert durch 1 oder 2 gleiche oder verschiedene Substituenten der Gruppe Hydroxy, Alkoxy, Halogen, Alkyl, Alkenyl, Aryl, Alkylmercapto, Alkylamino, Carboxy, Carbalkoxy, Cyano, Trifluormethyl, Trifluormethoxy oder Trifluormethylthio steht und

$R^4$ und $R^5$ gleich oder verschieden sind und jeweils für Wasserstoff oder für einen Alkyl-, Alkenyl- oder Alkinylrest stehen, wobei die Alkyl- und Alkenylreste geradkettig, verzweigt oder cyclisch sind und gegebenenfalls substituiert sind durch die Gruppe $COR^5$, wobei $R^5$ Alkyl, Aralkyl oder Aryl bedeutet,

oder

$R^4$ und $R^5$ gemeinsam mit dem Stickstoffatom einen 4- bis 7-gliedrigen Ring bilden, der gegebenenfalls durch Sauerstoff, Schwefel, die NH-Gruppe oder die $NR^6$-Gruppe unterbrochen ist, wobei $R^6$ die oben für $R^1$ und $R^2$ angegebene Bedeutung besitzt,

sowie ihre pharmazeutisch unbedenklichen Salze.

Es wurde gefunden, daß man Verbindungen der allgemeinen Formel (I) erhält, wenn man 2-Amino-3,4-dihydropyridinderivate der allgemeinen Formel (II)

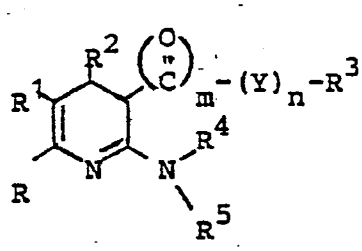

(II)

in welcher R, $R^1$, $R^2$, $R^3$, $R^4$, $R^5$ Y, m und n die oben angegebene Bedeutung haben,

in Gegenwart von inerten, organischen Lösungsmitteln und eines basischen Katalysators durch Sauerstoff zu den 2-Aminopyridin-derivaten (I) oxidiert.

Verwendet man 4,5-Bis-(4-methoxyphenyl)-6-methyl-2-(4-phenylpiperazino)-3,4-dihydropyridin, so kann der Reaktionsablauf durch folgendes Formelschema wiedergegeben werden:

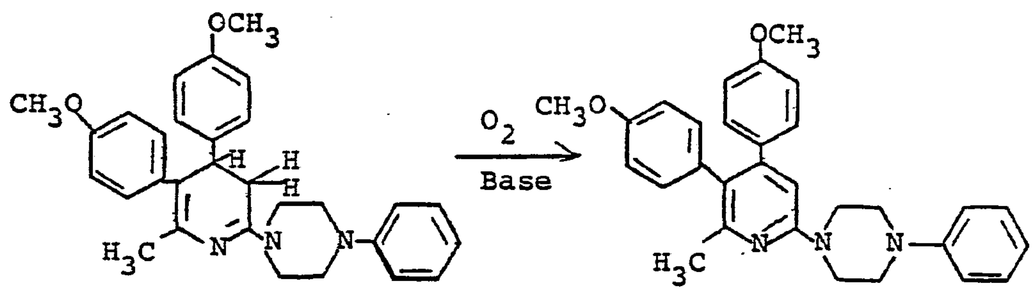


Die als Ausgangsverbindungen verwendbaren 2-Amino-3-4 dihydro-pyridinderivate der allgemeinen Formel (II) sind bekannt oder können nach bekannten Methoden hergestellt werden (vgl. DE-OS 2 949 701).

Bei der Durchführung des erfindungsgemäßen Verfahrens kommen als Verdünnungsmittel alle inerten organischen Lösungsmittel in Frage. Hierzu gehören vorzugsweise niedere Alkohole, wie Methanol, Ethanol, Propanol; Ether wie Tetrahydrofuran, Dioxan, Diethylether, 1,2-Dimethoxyethan; Ketone wie Aceton; Pyridin, Dimethylformamid, Dimethylsulfoxid, Hexamethylphosphorsäuretriamid oder Acetonitril.

Die Reaktionstemperaturen können in einem größeren Bereich variiert werden. Im allgemeinen arbeitet man

zwischen -80°C und 100°C, vorzugsweise zwischen -30°C bis 40°C.

Als basische Katalysatoren seien vorzugsweise genannt:

Alkali- und Erdalkalihydroxide und Alkoholate, insbesondere Alkalimetallalkoholate, Alkali- und Erdalkaliamide, insbesondere Alkalimetallamide, Alkali- und Erdalkalihydride, insbesondere Alkalimetallhydride, Alkali- und Erdalkalialkyl- und Alkali- und Erdalkaliarylverbindungen.

Die Umsetzung kann bei Normaldruck, aber auch bei erhöhtem Druck durchgeführt werden. Im allgemeinen arbeitet man bei Normaldruck.

Der für die Oxidation benötigte Sauerstoff kann in reiner Form oder verdünnt mit anderen, inerten Gasen, insbesondere Stickstoff oder Argon, zugeführt werden. Im allgemeinen wird Luftsauerstoff als Oxidationsmittel verwendet.

Wenn nicht ausdrücklich anders angegeben steht Alkyl in der vorliegenden Anmeldung für geradkettige, verzweigtes oder cyclisches Alkyl mit bis zu 10 Kohlenstoffatomen, insbesondere für geradkettiges oder verzweigtes Alkyl mit bis zu 6 Kohlenstoffatomen, Aryl bedeutet vorzugsweise Phenyl oder Naphthyl und Aralkyl steht vorzugsweise für Benzyl, Phenethyl oder Phenylpropyl. Alkoxy steht für geradkettiges oder verzweigtes Alkoxy mit 1 bis 6 Kohlenstoffatomen und Halogen bedeutet vorzugsweise Fluor, Chlor oder Brom.

Von besonderem Interesse sind Verbindungen der allgemeinen Formel (I), in welcher

R für geradkettiges oder verzweigtes Alkyl mit bis zu 6 Kohlenstoffatomen steht, welches gegebenenfalls 1- oder 2-fach substituiert ist durch Hydroxy, Alkoxy mit 1 bis 4 Kohlenstoffatomen, Amino, Monoalkylamino, Dialkylamino oder Benzyl-alkylamino, wobei die genannten Alkylgruppen 1 bis 4 Kohlenstoffatome enthalten oder für einen Phenyl- oder Benzylrest steht, wobei der Phenylring gegebenenfalls 1- oder 2-fach substituiert ist durch gleiche oder verschiedene Substituenten aus der Gruppe Fluor, Chlor, Brom, Alkyl, Alkoxy, Alkylmercapto mit jeweils 1 bis 4 Kohlenstoffatomen oder durch Trifluormethyl und

R und $R^1$ gemeinsam gegebenenfalls einen carbocyclischen Ring formen, an den ein Benzol- oder Naphthalinring ankondensiert ist, wobei der Benzol- bzw. Naphthalinring gegebenenfalls 1- oder 2-fach substituiert ist durch gleiche oder verschiedene Substituenten aus der Gruppe Fluor, Chlor, Brom, Alkyl, Alkoxy mit jeweils 1 bis 4 Kohlenstoffatomen oder durch Trifluormethyl, und der gegebenenfalls durch Sauerstoff, Schwefel, die Gruppe NH oder ein alkylsubstituiertes Stickstoffatom mit 1 bis 4 Kohlenstoffatomen unterbrochen ist.

$R^1$ und $R^2$ gleich oder verschieden sind und jeweils für geradkettiges oder verzweigtes Alkyl stehen, welches gegebenenfalls 1 oder 2 gleiche oder verschiedene Substituenten aus der Gruppe Phenyl, Hydroxy, Amino, Carboxy, Halogen, Alkylamino, Dialkylamino trägt,

oder

für Phenyl, Naphthyl, Furyl oder Pyridyl stehen, wobei diese aromatischen Ringe gegebenenfalls durch 1 oder 2 gleiche oder verschiedene Substituenten aus der Gruppe Nitro, Cyano, Azido, Hydroxy, Amino, Carboxy, Halogen, Phenyl, Alkyl, Alkoxy, Trifluormethyl, Trifluormethoxy, Benzoyloxy, Alkanoyloxy, Trifluormethylthio, Alkylmercapto, Alkylsulfonyl oder Alkylamino substituiert sind, wobei die genannten Alkyl- und Alkoxyreste jeweils 1 bis 4 Kohlenstoffatome enthalten, und ihrerseits gegebenenfalls substituiert sind durch Hydroxy, Alkoxy, Aroyloxy, Alkanoxyloxy oder die

Gruppe 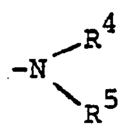

mit den unten angegebenen Bedeutungen für $R^4$ und $R^5$, und

m und n 0 oder 1 sein können und

Y für Sauerstoff, Schwefel oder die Gruppe NH steht, und

$R^3$ für Wasserstoff, geradkettiges oder verzweigtes Alkyl oder Alkenyl mit bis zu 6 Kohlenstoffatomen steht, gegebenenfalls substituiert durch Hydroxy, Alkoxy, Fluor, Chlor, Brom, Phenyl, Carboxy, Carbalkoxy oder die

Gruppe 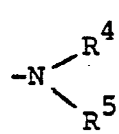

mit den unten angegebenen Bedeutungen für $R^4$ und $R^5$,

oder für Phenyl, Furyl, Pyridyl oder Naphtyl steht, wobei diese aromatischen Ringe gegebenenfalls 1 oder 2 gleiche oder verschiedene Substituenten aus der Gruppe Cyano, Hydroxy, Amino, Carboxy, Halogen, Phenyl, Alkyl, Alkoxy, Trifluormethyl, Trifluormethoxy, Trifluormethylthio, Alkylamino enthalten, und wobei die genannten Alkyl- und Alkoxyreste jeweils 1 bis 4 Kohlenstoffatome enthalten,

und

$R^4$ und $R^5$ gleich oder verschieden sind und jeweils für Wasserstoff, Alkyl oder Alkenyl mit bis zu 6 Kohlenstoffatomen stehen, wobei die Alkyl- und Benzylreste gegebenenfalls substituiert sind durch die Gruppe $COR^5$, wobei $R^5$ Alkyl mit 1 bis 4 Kohlenstoffatomen, Benzyl oder Phenyl bedeutet

oder

$R^3$ und $R^4$ gemeinsam mit dem Stickstoffatom einen 5- bis 7-gliedrigen Ring bilden, der gegebenenfalls durch Sauerstoff, Schwefel, NH oder $NR^6$ unterbrochen ist, wobei $R^6$ die für $R^1$ und $R^2$ angegebene Bedeutung besitzt.

Besonders hervorgehoben seien Verbindungen der allgemeinen Formel (I), in welcher

R für Alkyl mit 1 bis 4 Kohlenstoffatomen, Benzyl oder Phenyl steht,

$R^1$ und $R^2$ gleich oder verschieden sind und jeweils für Furyl, Pyridyl oder für Phenyl stehen, wobei der Phenylring gegebenenfalls 1 oder 2 gleiche oder verschiedene Substituenten aus der Gruppe Fluor, Chlor, Nitro, Cyano, Amino, Azido, Trifluormethyl, Trifluormethoxy, Trifluormethylthio, Alkylmercapto, Alkyl

mit 1 bis 4 Kohlenstoffatomen, Alkoxy mit 1 bis 4 Kohlenstoffatomen, Phenyl und Alkylamino mit 1 bis 2 Kohlenstoffatomen je Alkylrest, enthält,

m und n  O,

$R^3$  für Wasserstoff steht, und

$R^4$ und $R^5$  gleich oder verschieden sind und jeweils für Wasserstoff, Alkyl mit 1 bis 4 Kohlenstoffatomen stehen oder

$R^4$ und $R^5$  gemeinsam mit dem Stickstoffatom einen 5- oder 6-gliedrigen Ring bilden, der gegebenenfalls durch die Gruppe NH oder $NR^6$ unterbrochen ist, wobei $R^6$ die für $R^2$ angegebene Bedeutung besitzt.

Überraschenderweise zeigen die erfindungsgemäßen Verbindungen der Formel (I) eine sehr starke Wirkung bei der Behandlung von Fettstoffwechselstörungen. Sie bewirken insbesondere eine Senkung des erhöhten Cholesterins im Serum und vermindern gleichzeitig eine Hypertriglyceridämie.

Die erfindungsgemäßen Verbindungen eignen sich daher vorteilhaft zur Behandlung von Hyperlipoproteinämien, Atherosklerose, Adipositas und zur Behandlung hierdurch ausgelöster Stoffwechselstörungen.

Die erfindungsgemäßen Verbindungen sind besonders geeignet als Lipidabsorptionshemmer, Diuretika, Saluretika, Antiarrythmika und Cardiotonika.

Die diuretische und saluretische Wirkung wird an Ratten untersucht. Hierzu werden männliche Ratten verwendet, die in nüchternem Zustand 10 ml/kg Flüssigkeit per Schlundsonde erhalten. Diese Flüssigkeit enthält 0,5 Tylose sowie eine bestimmte Dosis des Prüfpräparates (Kontrolltiere ohne Prüfpräparat). Der ausgeschiedene Harn wird 6 Stunden gesammelt und anschließend der Natrium- und Kaliumgehalt in üblicher Weise photometrisch bestimmt.

Die antiarrythmische Wirkung der erfindungsgemäßen Verbindungen wird durch die Einflüsse auf die Refraktärzeit der Herzmuskulatur mittels Standardtestverfahren nachgewiesen. Bekanntlich verlängern Antiarrythmika in therapeutischen Dosen die Refraktärzeit der Herzmuskulatur. Diese Verlängerung der Refraktärzeit sowie die Bestimmung der Kontraktionskraft an isolierten Herzmuskel-Präparaten erfolgt nach bekannten Methoden (vgl.: Govier, J. Pharmacol. Exp. Ther. 148, 100-105, (1965) und Roseblueth et al., J. Cell Comp. Physiol. 33, 405-439 (1949)).

Aus der Stoffklasse der 2-Amino-pyridine sind bisher keine dieser Wirkungen bekannt geworden. Es ist daher als ausgesprochen überraschend zu bezeichnen, daß die erfindungsgemäßen Verbindungen diese neuen und vorteilhaften Wirkungen besitzen. Als neuartige Stoffklasse zur Behandlung von Stoffwechselstörungen und Herzrythmusstörungen, bei gleichzeitig sehr guter Verträglichkeit, stellen sie eine Bereicherung der Pharmazie dar.

Zur vorliegenden Erfindung gehören pharmazeutische Zubereitungen, die neben nichttoxischen, inerten pharmazeutisch geeigneten Trägerstoffen eine oder mehrere Verbindungen der obigen Formel enthalten oder die aus einer oder mehreren Verbindungen der obigen Formel bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen, vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosid entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, genannt.

Tabletten, Dragees, Kapseln, Pillen und Granulate können den oder die Wrikstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum-, Calcium- und Magensiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie don oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Lösungen und Emulsionen können neben dem oder den Wirkstoffen die üblichen Trägerstoffe, wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylenglykol, Suspendiermittel z.B. ethoxylierte Isostearylalkohole, Polyoxyethylensorbit- und sorbitanester, mikrokristalline Cellulose, Aluminiummethanhydroxid, Bentonit, Agar-Agar und Traganth oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können außer Verbindungen der obigen Formel auch andere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der Verbindungen der obigen Formel sowie die Verwendung von pharmazeutischen Zubereitungen, die eine oder mehrere Verbindungen der oben angegebenen Formel enthalten, in der Human- und Veterinärmedizin zur Verhütung, Besserung und/oder Heilung der oben angeführten Erkrankungen.

Die Wirkstoffe oder die pharmazeutischen Zubereitungen können oral, parenteral, intraperitoneal und/oder rektal, vorzugsweise oral, appliziert werden.

Im allgemeinen hat es sich sowohl in der Human- als auch in der Veterinärmedizin als vorteilhaft erwiesen, den oder die Wirkstoffe in Mengen von etwa 10 bis etwa 100 vorzugsweise 20 bis 100 mg/kg Körpergewicht je 24 Stunden, verteilt auf 1 bis 6 Verabreichungen und zwar vor oder/und während oder/ und nach der Mahlzeit zu applizieren. Eine Einzelgabe enthält den oder die Wirkstoffe, vorzugsweise in Mengen von etwa 10 bis etwa 100 mg/kg Körpergewicht. Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verarbreichung erfolgt. So kann es in einigen Fällen ausreichend sein, mit weniger als der o.g. Menge Wirkstoff auszukommen, während in anderen Fällen die oben angeführte Wirkstoffmenge überschritten werden muß.

Die Festlegung der jeweils erforderlichen optimalen Dosierung und Applikationsart der Wirkstoffe kann durch jeden Fachmann aufgrund seines Fachwissens leicht erfolgen.

Beispielhaft für die erfindungsgemäße Stoffgruppe sei die mit absorbtionshemmender Wirkung für die Verbindungen der Beispiele 1b und 5 durch die folgenden pharmakologischen Daten belegt.

| Beispiel Nr. | Dosis mg/kg | Verminderung des Lebercholesterin-anstiegs im Vergleich zur Kontrolle nach oraler Cholesterinbelastung in % |
|---|---|---|
| 1b | 100 | 27,6 % |
| | 30 | 10,7 % |
| 5 | 100 | 18,9 % |

## Beispiele

### Beispiel 1a

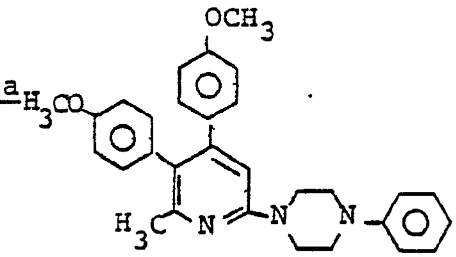

7 g (15 m Mol) 4,5-Bis-(4-methoxyphenyl)-6-methyl-2-(4-phenylpiperazino)-3,4-dihydropyridin werden in 150 ml DMSO p.a. unter Rühren suspendiert und mit 1.7 g (15 m Mol) Kalium tert. butylat versetzt. Es wird im verschlossenen Gefäß gerührt bis eine klare, organgerote Färbung entstanden ist. Dann wird die Reaktionslösung im offenen Reaktionskolben 1 h gerührt. Nach Neutralisation der Reaktionslösung mit Eisessig wird der ausgefallene, farblose Niederschlag abgesaugt, mit Methanol gewaschen und im Vakuum getrocknet.

Ausbeute 69 % der Theorie, Schmelzpunkt: 173 - 174°C.

### Beispiel 1b

2.1 g (4.5 m Mol) 4,5-Bis-(4-methoxyphenyl)-6-methyl-2-(4-phenylpiperazino)-pyridin werden in 200 ml absolutem Dichlormethan gelöst und auf -20°C bis -30°C gekühlt. Bei dieser Temperatur werden 19 ml einer Lösung von 100 ml Bortribromid in 300 ml Dichlormethan während 1h zugetropft.

Es wird 2h bei -20°C nachgerührt, dann werden langsam 30 ml Methanol zugetropft. Die Reaktionslösung wird mit ges. Natriumbicarbonatlösung gewaschen, mit Natriumsulfat getrocknet und im Vakuum eingedampft. Der so erhaltene ölige Rückstand kristallisiert aus Methanol.

Ausbeute: 84 % der Theorie, Schmelzpunkt: >230°C.

Die anderen in der nachfolgenden Tabelle aufgeführten Beispiele wurden analog zu Beispiel 1 hergestellt.

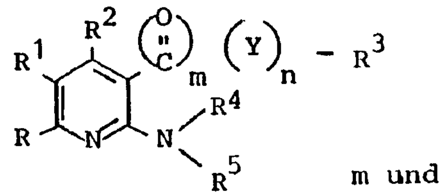

m und n = O und $R^3$ = H für alle Beispiele

| Beispiel Nr. | R | $R^1$ | $R^2$ | $R^3$ | $R^4$ | $R^5$ | Umkristallisiert aus Lösungsmittel | Fp. °C | Ausbeute in % der Theorie |
|---|---|---|---|---|---|---|---|---|---|
| 3 | $CH_3$ | 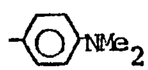 | 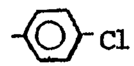 | H | $(-C_2H_4)_2N$- | 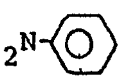 | Dimethylsulfoxid | 191-192 | 50 |
| 4 | $CH_3$ | 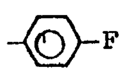 | 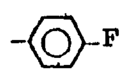 | H | $(-C_2H_4)_2N$- | 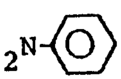 | Methanol | 155-156 | 57 |
| 5 | $CH_3$ | 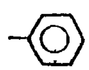 | 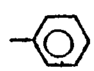 | H | $(-C_2H_4)_2N$- | 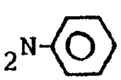 | Dimethylsulfoxid | 151-152 | 75 |
| 6 | $CH_3$ | 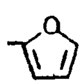 | 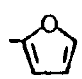 | H | $(-C_2H_4)_2N$- | 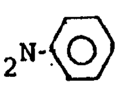 | Essigester/Ether x 2 HCl | 230 °C | 79 |
| 7 | $CH_3$ | 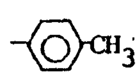 | 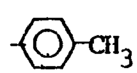 | H | $(CH_2)_4$-Pyrrolidin | | Aceton/Ether x 1 HCl | 205-206 | 72 |

Patentansprüche

1. 2-Amino-pyridinderivate der allgemeinen Formel (I)

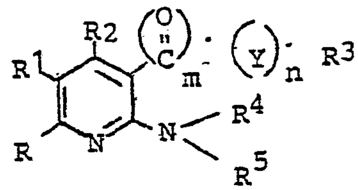

in welcher

R für Alkyl, gegebenenfalls substituiert durch Hydroxyl, Aryl oder Aralkyl bedeuten,

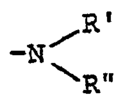

wobei R' und R" gleich oder verschieden sind und jeweils Wasserstoff, Alkyl oder Aralkyl bedeuten, steht
oder für gegebenenfalls substituiertes Aralkyl steht
oder für Aryl steht, welches gegebenenfalls durch 1 oder 2 gleiche oder verschiedene Substituenten aus der Gruppen Halogen, Alkyl, Alkoxy, Alkylmercapto oder Trifluormethyl substituiert ist,

und

R und $R^1$ gemeinsam gegebenenfalls einen carbocyclischen Ring formen, der gegebenenfalls substituiert ist durch Hydroxy, Halogen, Nitro, Alkoxy, die Gruppe

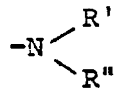 ,

wobei

R' und R" die oben angeführten Bedeutungen haben,

und an den ein Arylring ankondensiert sein kann, wobei der Arylring gegebenenfalls substituiert ist durch 1 oder 2 gleiche oder verschiedene Substituenten aus der Gruppe Halogen, Alkyl, Alkoxy, Alkylmercapto oder Trifluormethyl,
und der gegebenenfalls durch Sauerstoff, Schwefel oder die $NR^6$-Gruppe unterbrochen ist wobei $R^6$ die für $R^1$ und $R^2$ angegebene Bedeutung besitzt, und

$R^1$ und $R^2$ gleich oder verschieden sind und jeweils für Alkyl, gegebenenfalls substituiert durch Aryl, Heteroaryl, Hydroxyl, Halogen, Alkoxy, Carboxy, Cyano, Carbalkoxy oder die Gruppe

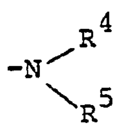

mit den unten angeführten Bedeutungen für $R^4$ und $R^5$ stehen,

oder

für einen Arylrest oder für einen Heteroarylrest aus der Gruppe Thienyl, Furyl, Pyrryl, Pyrazolyl, Imidazolyl, Oxazolyl, Isooxazolyl, Thiazolyl, Pyridyl,

Pyridazinyl, Pyrimidyl, Pyrazinyl, Chinolyl, Isochinolyl, Indolyl, Benzimidazolyl, Chinazolyl oder Chinoxalyl stehen, wobei der Arylrest sowie die Heteroarylreste gegebenenfalls 1, 2 oder 3 gleiche oder verschiedene Substituenten aus der Gruppe Phenyl, Alkyl, Alkenyl, Alkinyl, Alkoxy, Alkenoxy, Alkinoxy, Alkylen, Dioxyalkylen, Halogen, Acyloxy, Trifluormethylthio, Trifluormethyl, Trifluormethoxy, Hydroxy, die

Gruppe 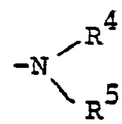

mit den unten angegebenen Bedeutungen für $R^4$ und $R^5$, Nitro, Cyano, Azido, Carboxy, Carbalkoxy, Carbonamido, Sulfonamido oder $SO_m$-Alkyl (m = 0, 1 oder 2) enthalten, und wobei die Alkyl- und Alkoxysubstituenten ihrerseits gegebenenfalls substituiert sind durch Hydroxy, Alkoxy, Carboxy, Carbalkoxy, Halogen, die Gruppe

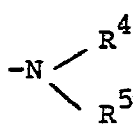 , mit den unten angegebenen Bedeutungen für $R^4$ und $R^5$, Aroyloxy oder Alkanoyloxy, wobei die Aroyl- und Alkanoylreste ihrerseits gegebenenfalls durch Hydroxy, Alkoxy, Halogen, Carboxy oder Carbalkoxy substituiert sind, und

m und n gleich oder verschieden sind und jeweils 0 oder 1 bedeuten, und

Y für Sauerstoff, die Gruppe $SO_{m'}$ ($m' = 0,1,2$) oder die Gruppe $N\text{-}R^6$, wobei $R^6$ einen Alkyl- oder Arylrest bedeutet, steht und

$R^3$ für Wasserstoff oder Alkyl, gegebenenfalls substituiert durch Hydroxy, Epoxy, Alkoxy, Halogen oder die

Gruppe 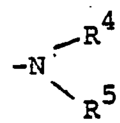

mit den unten angeführten Bedeutungen für $R^4$ und $R^5$ steht, oder für Carboxy, Carbalkoxy, Cyano, Alkenyl, Aryl oder für Aryl, gegebenenfalls substituiert durch 1 oder 2 gleiche oder verschiedene Substituenten der Gruppe Hydroxy, Alkoxy, Halogen, Alkyl, Alkenyl, Aryl, Alkylmercapto, Alkylamino, Carboxy, Carbalkoxy, Cyano, Trifluormethyl, Trifluormethoxy oder Trifluormethylthio steht und

$R^4$ und $R^5$ gleich oder verschieden sind und jeweils für Wasserstoff oder für einen Alkyl-, Alkenyl- oder Alkinylrest stehen, wobei die Alkyl- und Alkenylreste geradkettig, verzweigt oder cyclisch sind und gegebenenfalls substituiert sind durch die Gruppe $COR^5$, wobei $R^5$ Alkyl, Aralkyl oder Aryl bedeutet,

oder

$R^4$ und $R^5$ gemeinsam mit dem Stickstoffatom einen 4- bis 7-gliedrigen Ring bilden, der gegebenenfalls durch Sauerstoff, Schwefel, die NH-Gruppe oder die $NR^6$-Gruppe unterbrochen ist, wobei $R^6$ die oben für $R^1$ und $R^2$ angegebene Bedeutung besitzt,

sowie ihre pharmazeutisch unbedenklichen Salze.

2. Verbindungen der allgemeinen Formel (I) mit Anspruch 1 in welcher

R für geradkettiges oder verzweigtes Alkyl mit bis zu 6 Kohlenstoffatomen steht, welches gegebenenfalls 1- oder 2-fach substituiert ist durch Hydroxy, Alkoxy mit 1 bis 4 Kohlenstoffatomen, Amino, Monoalkylamino, Dialkylamino oder Benzyl-alkylamino, wobei die genannten Alkylgruppen 1 bis 4 Kohlenstoffatome enthalten oder für einen Phenyl- oder Benzylrest steht, wobei der Phenylring gegebenenfalls 1- oder 2-fach substituiert ist durch gleiche oder verschiedene Substituenten aus der Gruppe Fluor, Chlor, Brom, Alkyl, Alkoxy, Alkylmercapto mit jeweils 1 bis 4 Kohlenstoffatomen oder durch Trifluormethyl

und

R und $R^1$ gemeinsam gegebenenfalls einen carbocyclischen Ring formen, an den ein Benzol- oder Naphthalinring ankondensiert ist, wobei der Benzol- bzw. Naphthalinring gegebenenfalls 1- oder 2-fach substituiert ist durch gleiche oder verschiedene Substituenten aus der Gruppe Fluor, Chlor, Brom, Alkyl, Alkoxy mit jeweils 1 bis 4 Kohlenstoffatomen oder durch Trifluormethyl, und der gegebenenfalls durch Sauerstoff, Schwefel, die Gruppe NH oder ein alkylsubstituiertes Stickstoffatom mit 1 bis 4 Kohlenstoffatomen unterbrochen ist,

$R^1$ und $R^2$ gleich oder verschieden sind und jeweils für geradkettiges oder verzweigtes Alkyl stehen, welches gegebenenfalls 1 oder 2 gleiche oder verschiedene Substituenten aus der Gruppe Phenyl, Hydroxy, Amino, Carboxy, Halogen, Alkylamino, Dialkylamino trägt,

oder

für Phenyl, Naphthyl, Furyl oder Pyridyl stehen, wobei diese aromatischen Ringe gegebenenfalls durch 1 oder 2 gleiche oder verschiedene Substituenten aus der Gruppe Nitro, Cyano, Azido, Hydroxy, Amino, Carboxy, Halogen, Phenyl, Alkyl, Alkoxy, Trifluormethyl, Trifluormethoxy, Benzoyloxy, Alkanoyloxy, Trifluormethylthio, Alkylmercapto, Alkylsulfonyl oder Alkylamino substituiert sind, wobei die genannten Alkyl- und Alkoxyreste jeweils 1 bis 4 Kohlenstoffatome enthalten, und ihrerseits gegebenenfalls substituiert sind durch Hydroxy, Alkoxy, Aroyloxy, Alkanoxyloxy oder die

Gruppe 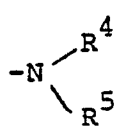

mit den unten angegebenen Bedeutungen für $R^4$ und $R^5$, und

m und n 0 oder 1 sein können und

Y für Sauerstoff, Schwefel oder die Gruppe NH steht, und

$R^3$ für Wasserstoff, geradkettiges oder verzweigtes Alkyl oder Alkenyl mit bis zu 6 Kohlenstoffatomen steht, gegebenenfalls substituiert durch Hydroxy, Alkoxy, Fluor, Chlor, Brom, Phenyl, Carboxy, Carbalkoxy oder die

Gruppe 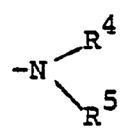

mit den unten angegebenen Bedeutungen für $R^4$ und $R^5$,

oder für Phenyl, Furyl, Pyridyl oder Naphtyl steht, wobei diese aromatischen Ringe gegebenenfalls 1 oder 2 gleiche oder verschiedene Substituenten aus der Gruppe Cyano, Hydroxy, Amino, Carboxy, Halogen, Phenyl, Alkyl, Alkoxy, Trifluormethyl, Trifluormethoxy, Trifluormethylthio, Alkylamino enthalten, und wobei die genannten Alkyl- und Alkoxyreste jeweils 1 bis 4 Kohlenstoffatome enthalten,

und

$R^4$ und $R^5$ gleich oder verschieden sind und jeweils für Wasserstoff, Alkyl oder Alkenyl mit bis zu 6 Kohlenstoffatomen stehen, wobei die Alkyl- und Benzylreste gegebenenfalls substituiert sind durch die Gruppe $COR^5$, wobei $R^5$ Alkyl mit 1 bis 4 Kohlenstoffatomen, Benzyl oder Phenyl bedeutet

oder

$R^3$ und $R^4$ gemeinsam mit dem Stickstoffatom einen 5- bis 7-gliedrigen Ring bilden, der gegebenenfalls durch Sauerstoff, Schwefel, NH oder $NR^6$ unterbrochen ist, wobei $R^6$ die für $R^1$ und $R^2$ angegebene Bedeutung besitzt.

3. Verbindungen der allgemeinen Formel (I) gemäß Anspruch 1 in welcher

R für Alkyl mit 1 bis 4 Kohlenstoffatomen, Benzyl oder Phenyl steht,

$R^1$ und $R^2$ gleich oder verschieden sind und jeweils für Furyl, Pyridyl oder für Phenyl stehen, wobei der Phenylring gegebenenfalls 1 oder 2 gleiche oder verschiedene Substituenten aus der Gruppe Fluor, Chlor, Nitro, Cyano, Amino, Azido, Trifluormethyl, Trifluormethoxy, Trifluormethylthio, Alkylmercapto, Alkyl mit 1 bis 4 Kohlenstoffatomen, Alkoxy mit 1 bis 4 Kohlenstoffatomen, Phenyl und Alkylamino mit 1 bis 2 Kohlenstoffatomen je Alkylrest, enthält,

m und n 0,

$R^3$ für Wasserstoff steht, und

$R^4$ und $R^5$ gleich oder verschieden sind und jeweils für Wasserstoff, Alkyl mit 1 bis 4 Kohlenstoffatomen stehen oder

$R^4$ und $R^5$ gemeinsam mit dem Stickstoffatomen einen 5- oder 6-gliedrigen Ring bilden, der gegebenenfalls durch die Gruppe NH oder $NR^6$ unterbrochen ist, wobei $R^6$ die für $R^2$ angegebene Bedeutung besitzt.

4. Verfahren zur Herstellung von Verbindungen der allgemeinen Formel (I)

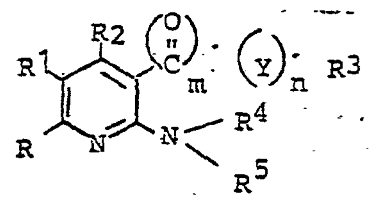

in welcher

R für Alkyl, gegebenenfalls substituiert durch Hydroxyl, Alkyl oder Aralkyl bedeuten,

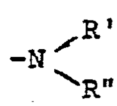

wobei R' und R" gleich oder verschieden sind und jeweils Wasserstoff, Alkyl oder Aralkyl bedeuten, steht

oder für gegebenenfalls substituiertes Aralkyl steht
oder für Aryl steht, welches gegebenenfalls durch 1 oder 2 gleiche oder verschiedene Substituenten aus der Gruppen Halogen, Alkyl, Alkoxy, Alkylmercapto oder Trifluormethyl substituiert ist,

und

R und $R^1$ gemeinsam gegebenenfalls einen carbocyclischen Ring formen, der gegebenenfalls substituiert ist durch Hydroxy, Halogen, Nitro, Alkoxy, die Gruppe

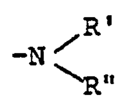 ,

wobei

R' und R" die oben angeführten Bedeutungen haben,

und an den ein Arylring ankondensiert sein kann, wobei der Arylring gegebenenfalls substituiert ist durch 1 oder 2 gleiche oder verschiedene Substituenten aus der Gruppe Halogen, Alkyl, Alkoxy, Alkylmercapto oder Trifluormethyl,
und der gegebenenfalls durch Sauerstoff, Schwefel oder die $NR^6$-Gruppe unterbrochen ist wobei $R^6$ die für $R^1$ und $R^2$ angegebene Bedeutung besitzt, und

$R^1$ und $R^2$ gleich oder verschieden sind und jeweils für Alkyl, gegebenenfalls substituiert durch Aryl, Heteroaryl, Hydroxyl, Halogen, Alkoxy, Carboxy, Cyano, Carbalkoxy oder die Gruppe

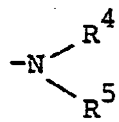

mit den unten angeführten Bedeutungen für $R^4$ und $R^5$ stehen,

oder

für einen Arylrest oder für einen Heteroarylrest aus der Gruppe Thienyl, Furyl, Pyrryl, Pyrazolyl, Imidazolyl, Oxazolyl, Isooxazolyl, Thiazolyl, Pyridyl, Pyridazinyl, Pyrimidyl, Pyrazinyl, Chinolyl, Isochinolyl, Indolyl, Benzimidazolyl, Chinazolyl oder Chinoxalyl stehen, wobei der Arylrest sowie die Heteroarylreste gegebenenfalls 1, 2 oder 3 gleiche oder verschiedene Substituenten aus der Gruppe Phenyl, Alkyl, Alkenyl, Alkinyl, Alkoxy, Alkenoxy, Alkinoxy, Alkylen, Dioxyalkylen, Halogen, Acyloxy, Trifluormethylthio, Trifluormethyl, Trifluormethoxy, Hydroxy, die Gruppe 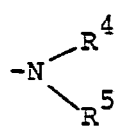

mit den unten angegebenen Bedeutungen für $R^4$ und $R^5$, Nitro, Cyano, Azido, Carboxy, Carbalkoxy, Carbonamido,

0068259

Sulfonamido oder $SO_m$-Alkyl ($m = 0, 1$ oder $2$) enthalten, und wobei die Alkyl- und Alkoxysubstituenten ihrerseits gegebenenfalls substituiert sind durch Hydroxy, Alkoxy, Carboxy, Carbalkoxy, Halogen, die Gruppe

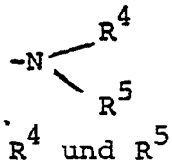 , mit den unten angegebenen Bedeutungen für

$R^4$ und $R^5$, Aroyloxy oder Alkanoyloxy, wobei die Aroyl- und Alkanoylreste ihrerseits gegebenenfalls durch Hydroxy, Alkoxy, Halogen, Carboxy oder Carbalkoxy substituiert sind, und

m und n gleich oder verschieden sind und jeweils 10 oder 1 bedeuten, und

Y für Sauerstoff, die Gruppe $SO_{m'}$ ($m' = 0,1,2$) oder die Gruppe $N-R^6$, wobei $R^6$ einen Alkyl- oder Arylrest bedeutet, steht und

$R^3$ für Wasserstoff oder Alkyl, gegebenenfalls substituiert durch Hydroxy, Epoxy, Alkoxy, Halogen oder die

Gruppe 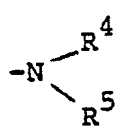

mit den unten angeführten Bedeutungen für $R^4$ und $R^5$ steht, oder für Carboxy, Carbalkoxy, Cyano, Alkenyl, Aryl oder für Aryl, gegebenenfalls

substituiert durch 1 oder 2 gleiche oder verschiedene Substituenten der Gruppe Hydroxy, Alkoxy, Halogen, Alkyl, Alkenyl, Aryl, Alkylmercapto, Alkylamino, Carboxy, Carbalkoxy, Cyano, Trifluormethyl, Trifluormethoxy oder Trifluormethylthio steht und

$R^4$ und $R^5$ gleich oder verschieden sind und jeweils für Wasserstoff oder für einen Alkyl-, Alkenyl- oder Alkinylrest stehen, wobei die Alkyl- und Alkenylreste geradkettig, verzweigt oder cyclisch sind und gegebenenfalls substituiert sind durch die Gruppe $COR^5$, wobei $R^5$ Alkyl, Aralkyl oder Aryl bedeutet,

oder

$R^4$ und $R^5$ gemeinsam mit dem Stickstoffatom einen 4- bis 7-gliedrigen Ring bilden, der gegebenenfalls durch Sauerstoff, Schwefel, die NH-Gruppe oder die $NR^6$-Gruppe unterbrochen ist, wobei $R^6$ die oben für $R^1$ und $R^2$ angegebene Bedeutung besitzt,

sowie ihre pharmazeutisch unbedenklichen Salze, dadurch gekennzeichnet, daß man 2-Amino-3,4-dihydropyridinderivate der allgemeinen Formel (II)

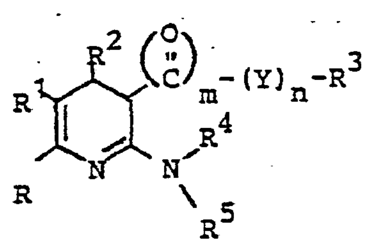

in welcher

$R^1, R^2, R^3, R^4, R^5, Y,$ m und n die oben angegebene Bedeutung haben,

in Gegenwart von inerten, organischen Lösungsmitteln und eines basischen Katalysators durch Sauerstoff zu den 2-Aminopyridin-derivaten (I) oxidiert.

5. Verfahren gemäß Anspruch 4, dadurch gekennzeichnet, daß man die Reaktion bei Temperaturen zwischen -80°C und +100°C durchführt und als basische Katalysatoren Alkali- oder Erdalkali enthaltende Basen verwendet.

6. Verbindungen der allgemeinen Formel (I) gemäß Anspruch 1 zur Verwendung bei der Bekämpfung von Erkrankungen.

7. Verbindungen der allgemeinen Formel (I) gemäß Anspruch 1 zur Verwendung bei der Bekämpfung von Erkrankungen des Fettstoffwechsels.

8. Arzneimittel enthaltend mindestens einer Verbindung der allgemeinen Formel (I) gemäß Anspruch 1.

9. Verfahren zur Herstellung von Arzneimittel, dadurch gekennzeichnet, daß man mindestens eine Verbindung der allgemeinen Formel (I) gemäß Anspruch 1 gegebenenfalls unter Verwendung von Hilfs- und Trägerstoffen in eine geeignete Applikationsform überführt.

10. Verwendung von Verbindungen der allgemeinen Formel (I) gemäß Anspruch 1 bei der Bekämpfung von Erkrankungen.

Europäisches Patentamt

**EUROPÄISCHER RECHERCHENBERICHT**

0068259

Nummer der Anmeldung
EP 82 10 5170

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP-A-0 000 816 (BEECHAM GROUP LTD.) * Ansprüche * | 1,8 | C 07 D 213/74<br>C 07 D 407/04<br>A 61 K 31/44 |
| A | DE-A-2 230 392 (CASSELLA FARBWERKE MAINKUR AG) * Ansprüche * | 1 | |
| D,A | DE-A-2 949 701 (BAYER AG) * Ansprüche * | 1,8 | |
| D,A | EP-A-0 013 153 (BEECHAM GROUP LTD.) * Ansprüche * | 1,8 | |
| | | | **RECHERCHIERTE SACHGEBIETE (Int. Cl. 3)** |
| | | | C 07 D 213/00<br>C 07 D 405/00 |

Der vorliegende Recherchenbericht wurde für alle Patentansprüche erstellt.

| Recherchenort | Abschlußdatum der Recherche | Prüfer |
|---|---|---|
| DEN HAAG | 20-09-1982 | VAN BIJLEN H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82